# EUROPÄISCHE PATENTSCHRIFT

(11) **EP 1 175 877 B1**
(45) Veröffentlichungstag und Bekanntmachung des Hinweises auf die Patenterteilung: **26.09.2007**
(21) Anmeldenummer: 01118347.2
(22) Anmeldetag: 27.07.2001
(51) Int. Cl.: A61F 2/36

(54) **Modulare Revisionsprothese**
Modular revision prosthesis
Prothèse de révision modulaire

(30) Priorität: 29.07.2000 DE 10036984
(43) Veröffentlichungstag der Anmeldung: 30.01.2002
(73) Patentinhaber: Thurgauer Kantonalbank, 8570 Weinfelden (CH)
(72) Erfinder: Draenert, Klaus, 81545 München (DE)
(74) Vertreter: Rentsch & Partner

(56) Entgegenhaltungen:
- EP-A- 0 496 089
- EP-A- 0 592 897
- EP-A- 0 634 153
- WO-A-90/02533
- DE-A- 4 234 351
- DE-C- 19 610 741
- FR-A- 2 799 115
- US-A- 5 376 126
- US-A- 5 658 333
- US-A- 5 725 592

## Beschreibung

### Hintergrund der Erfindung

Die Wiederherstellung der Funktion von einem gelockerten, künstlichen Hüftgelenk setzt voraus, daß große und kleinere Probleme gemeistert wenden. Die großen Probleme sind die Verankerungsprobleme einer stabilen Fixation bei den oft großen Defekten, die im knöchernen Lager zurückbleiben, nachdem die Gelenkkomponenten entfernt worden sind. Kleinere Probleme sind die Auffüllung der Defekte mit Bankknochen; dies gelingt mit "morcellized bone"-Plastiken entsprechender Größe (Lamerigts NMP, 1998 Proefschrift an der katholischen Universität Niymegen). Ist das knöcherne Lager wieder durch Bankknochen verstärkt worden, so kann eine entsprechende Gelenkersatzkomponente einzementiert werden.

Die Voraussetzungen für ein solches Vorgehen sind dabei genügend stabile Knochenstrukturen, die eine stabile Gesamtverankerung gewährleisten. Sehr oft sind diese Strukturen jedoch nicht mehr vorhanden, was ganz besondere Anforderungen an das Implantat stellt. Es besteht daher ein echter Bedarf an Systemen, die den jeweiligen Verhältnissen angepaßt werden können und verschiedene biomechanische Fixationsprinzipien berücksichtigen. Auf dem Boden dieser Erkenntnisse konnte überraschend einfach ein neuer Zugang zum Problem der Revisionsoperationen gefunden werden.

### Stand der Forschung

Die Defekte im knöchernen Femurbett nach der Entfernung einer gelockerten Prothese können unterschiedliche Ausmaße erreichen, was zu Klassifikationen der Knochendefekte geführt hat, beispielsweise in der DGOT (Bettin, D., Katthagen, B. D. (1997) Die DGOT-Klassifikation von Knochendefekten bei Hüft-Totalendoprothesen-Revisionsoperationen Z. Orthop. 135). Teilsweise sind die Knochenzerstörungen erheblich. Die Behandlung der gelockerten Komponenten besteht in der vollständigen Entfernung der Komponenten und gegebenenfalls des Knochenzementes und allen das Implantat umgebenden Bindegewebes. Erst dann kann das Ausmaß des Knochchenverlustes realistisch beurteilt werden. Die Verankerung einer neuen Komponente ist oft nur dadurch möglich, daß alle Defekte überbrückt werden und tief in der noch gesunden Femurdiaphyse verankert werden, und zwar sehr oft zementfrei. So ist in der US PA 5,725,592 eine modulare, zementfreie Prothese beschrieben, die für Primäroperationen zur Anwendung kommt, und die bei Revisionen, v.a. proximal nicht genügend Verankerungsmöglichkeiten hat.

Eine andere Methode ist der Wiederaufbau des Knochens mit zerkleinertem Bankknochen und das erneute Einzementieren einer solchen Komponente, übersichtlich beschrieben in Lamerigts, N. M. (1998) The Incorporative Process of Morcellized Bone Graft. Proefschrift University Nijmegen (Katholische Universität). In beiden Fällen ist die Verankerung proximal meist nicht stabil, sind die Implantate meist sehr lang und schwer und die Ergebnisse deutlich schlechter wie die von Primäroperationen.

Untersuchungen und einfache Versuche am Leichenknochen zeigten überraschend sehr effiziente Möglichkeiten, um Femurkomponenten auch mit kürzeren Schäften sehr stabil in defekten Knochenköcher zu verankern und zu fixieren.

### Beschreibung der Erfindung

Das modulare zuggurtungssystem ermöglicht die Anpassung an verschiedene Defektzustände bei der Revision einer gelockerten Femurkomponente. Die Erfindung berücksichtigt dabei verschiedene zu überbrückende Defekte in Bezug auf die Stiel- oder Schaftlänge und verschiedene zusätzliche Verankerungsmöglichkeiten im proximalen Femurköcher. Das Modulare System besteht im wesentlichen aus einer Achse (100), die einem Zylinder um die Markkanalachse entspricht. Um diesen Zylinder sind verschiedene Segmente aufzureihen: Das Basissegment (100.1) kann verschieden lang sein und besteht immer aus der Spitze (102) und dem Achsenzylinder (103). Über dem Achsenzylinder können ein, selten zwei oder mehr (1002, 100.3) Stielsegmente aufgefädeet werden, denen abschließend immer ein Basissegment folgt (100.1). Die Aufnahmefläche (105) am Basissegment ist konkav, das korrespondierende Ende vom Mittelsegment ist konvex gestaltet oder umgekehrt, beide artikulierenden Enden können auch konisch ineinandergreifen, besonders bewährt hat sich eine geschwungene, ineinandergreifende Oberflächengestaltung, die eine Verdrehung sichern und Zugkräfte auf der lateralen und Druckkräfte auf der medialen Seite berücksichtigt.

Der Achsenzylinder (103) und die korrespondierende Bohrung sind glatt oder mit einer Führungsrille und Noppen strukturiert, im Sinne einer Verdrehsicherung. Je nach Defektüberbrückung wird die Lange der Prothese bestimmt und über dem zentralen Achsenzylinder (103) ein Mittelsegment (100.2) über die Bohrung (106) aufgesetzt. Der Querschnitt (108) des Stieles besteht aus dem lateralen Zylinder, welcher auch hohl sein kann (109 und 112), dem Verbindungssegment (111) und der medialen Portion (110) und bildet die konvex-konkav (104)-konvexe Kontur der Dorsalseite. Die mediale Portion ist Ober weite Bereiche vom Kanal des Zugankers (Schubstange) durchzogen (113).

Das metaphysäre Segment zeigt im U-Shape der Kraftübertragung, medial, ventral und dorsal eine parabelförmig gekrümmte Mantelfläche. Die Schulter (200) der Prothese weist weitere Bohrungen auf für Zuggurtungen (60) und Seilzüge (71). Im Konus der Prothese (300) wird der Schubanker (50) aufgenommen, der axial durch den Konus im Sinne einer Zugschraube oder, wie abgebildet, mit Unterlegscheibe (54) und Schraubenkopf (51), sowie einer verdrehgesicherten Mutter (55). Auch die übrigen Zuganker können als einfache Zugschrauben ausgeführt werden (z. B. 60), wobei der Schraubenkopf dann in der Schulter_{.} (200) untergebracht wäre und das Zugschraubengewinde distal im Knochen.

Durch die Kombination von Zuganker, Zugschrauben und Seilzügen können die Prothesenstiele selbst in Knochenköchern mit großen Defekten stabil fixiert werden.

### Beispiel

Nach eindeutiger Diagnosestellung einer Hüftprothesenlockerung wird das Gelenk über den alten Zugang freigelegt; das Narbengewebe wird sorgfältig entfernt, das Gelenk luxiert und der gelockerte Schaft entfernt. Meist läßt er sich einfach herausziehen, selten ist ein Ausschlaginstrument notwendig. Der Knochenzement und das Bindegewebe werden daraufhin sorgfältig herauspräpariert, wobei ein Ultraschalititanmeissel dabei sehr hilfreich sein kann.

Der Knochenköcher, der von Bindegewebe befreit worden ist, wird kräftig mit der Jet-Lavage gespült und anschließend knöchern wiederaufgebaut; hierzu benützt man Bankknochen, der in einer Mühle zerkleinert wurde, dieser sogenannte morcellized bone wird 50:50 mit einer schalenförmigen Knochenkeramik als Granulat, z. B. Synthacer^{®}, vermischt und mit Hilfe eines Probeschaftes in der Markhöhle wandständig komprimiert. Danach werden Drainageröhrchen über die fossa intertrochanterica eingeführt und an diese ein Vakuum angelegt. Im Anschluß daran wird die Markhöhle vorsichtig mit H₂O₂ gewaschen und mit Knochenzement aufgefüllt, wofür ein Schnorchelapplikationssystem verwendet wird. In das mit Knochenzement un Knochen aufgefüllte Bett wird die montiert Prothese axial eingeführt. Nath Aushärten des Zementes wird die Prothese konusaxial und wenn notwendig durch weitere vorgegebene Bohrungen gebohrt und mit Zugankern oder Zugschrauben im Knochen des proximalen Köchers stabil verankert. Die Schrauben lassen sich auch vorteilhaft durch den noch weichen Zement eindrehen, wenn die Bohrungen im Knochen vorgebohrt wurden. Dies hat den Vorteil, daß der Knochenzement auf das Schraubengewinde aufschrumpft.

Bei noch stabilen Knochenverhältnissen kann das Prothesensystem auch ohne Knochenzement stabil im Femurköcher verankert werden.

### Modulare Revisionsprothese

| | |
|---|---|
| Abb. 01 | Modularsystem NPS in ein Femur projiziert. |
| Abb. 01/00 | Stielsegmente |
| Abb. 01/100.1 | Metaphysäres Segment |
| Abb. 01/100.2 | Diaphysäres Mittelsegment |
| Abb.01/100.3 | Basissegment |
| Abb. 01/101 | Offset = Markkanalachse-Rotationszentrum |
| Abb. 01/102 | Spitze des Basissegmentes |
| Abb. 01/103 | Achsenzylinder des Basissegmentes |
| Abb. 01/105 | Aufnahmefläche der Segmente (Basissegment und Stielsegment) |
| Abb. 01/106 | Bohrung des Stielsegmentes |
| Abb. 01/110 | Eröffnungsbohrung |
| Abb. 01/130 | Markkanalachse |
| Abb. 01/200 | Schulter der Prothese |
| Abb. 10/300 | Konus der Prothese |
| | |
| Abb. 01/50 | Schubanker |
| Abb. 01/51 | Schraubenkopf |
| Abb. 01/54 | Unterlegscheibe |
| Abb. 01/55 | Konus Mutter |
| Abb. 01/60 | Zugankerschraube |
| Abb. 01/70 | Zuggurtung |
| Abb. 01/71 | Bohrung in der Schulter der Prothese für Zuggurtung |
| | |
| Abb. 02 | |
| Abb. 02/104 | Übergangssegment zwischen lateraler Portion und medialer Portion des Prothesenquerschnittes im metaphysären Segment |
| Abb. 02/108 | Konvex-konkav-konvexe Schwingung der dorsalen Mantelfläche |
| Abb. 02/109 | Metaphysäres Segment, laterale Portion |
| Abb. 02/110 | Metaphysäres Segment, mediale Portion |
| Abb. 02/111 | Konvexe Krümmung der ventralen Mantelfläche |
| Abb. 02/112 | Bohrung entlang der Kanalachse im lateralen Segment |
| Abb. 02/113 | Schubankerbohrkanal |
| | |
| Abb. 03 | Modularsystem NPS in ein Femur projiziert. |
| Abb. 03/100 | Stielsegmente |
| Abb. 03/100.1 | Metaphysäres Segment |
| Abb. 03/100.3 | Basissegment |
| Abb. 03/101 | Offset = Markkanalachse-Rotationszentrum |
| Abb. 03/102 | Spitze des Basissegmentes |
| Abb. 03/103 | Achsenzylinder des Basissegmentes |
| Abb. 03/105 | Aufnahmefläche der Segmente (Basissegment und Stielsegment) |
| Abb. 03/106 | Bohrung des Stielsegmentes |
| Abb. 03/110 | Eröffnungsbohrung |
| Abb. 03/130 | Markkanalachse |
| Abb. 03/200 | Schulter der Prothese |
| Abb. 03/300 | Konus der Prothese |
| | |
| Abb. 03/50 | Schubanker |
| Abb. 03/51 | Schraubenkopf |
| Abb. 03/54 | Unterlegscheibe |
| Abb. 03/55 | Konus Mutter |
| Abb. 03/60 | Zugankerschraube |
| Abb. 03/70 | Zuggurtung |
| Abb. 03/71 | Bohrung in der Schulter der Prothese für Zuggurtung |

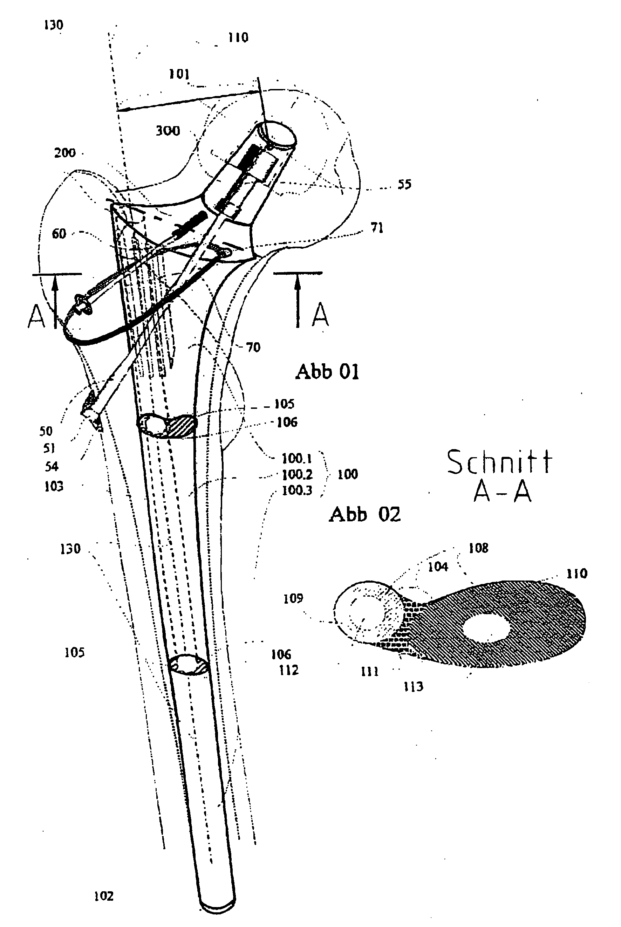

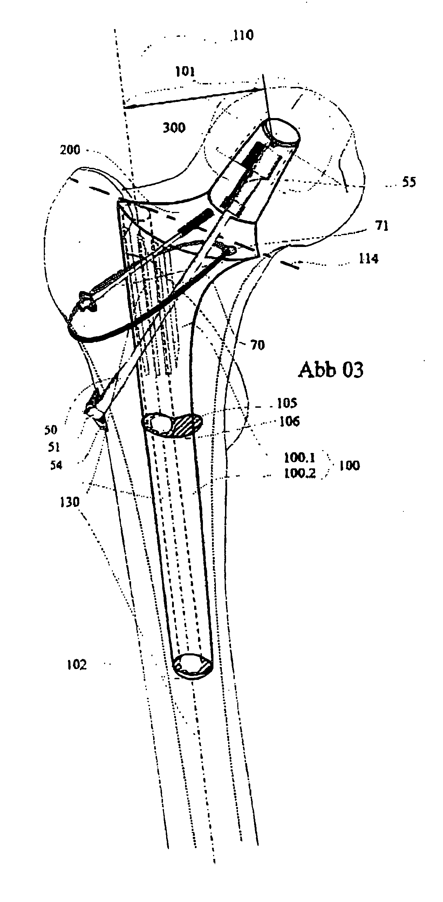

## Patentansprüche

1. Femurkomponente eines künstlichen Hüftgelenkes für die zementfreie oder zementierte Revisionsoperation oder Primäroperation, umfassend einen
Stiel der ein- oder mehrteilig ausgebildet ist, wobei
der Stiel aus ein dorsolaterales zylindrisches Konstruktionselement besitzt, welches axial zur Markkanalachse (130) ausgerichtet ist, massiv oder durchbohrt sein kann und fest oder montierbar mit einem proximalen anatomischen metaphysären Segment (100.1) verbunden ist, welches kegelförmig medial exzentrisch aufgebaut ist, wobei
das proximale metaphysäre Segment (100.1) über einer Schulter (200) den Konus (300) für die zentrische oder exzentrische Kopfaufnahme trägt, **dadurch gekennzeichnet, dass** die Schulter (200) eine oder mehrere Bohrungen (71) ***für die Aufnahme einer Zuggurtung*** (70) aufweist.

2. Femurkomponente nach Anspruch (1) so ausgebildet, daß das zylindrische konstruktionselement (103) als durchgehende Konstruktionsachse dorsal und lateral das im Querschnitt nierenförmige metaphysäre Segment über eine vollständig oder nicht geschlossene Bohrung aufnimmt und dieses ein- oder mehrteilig ausgebildet ist.

3. Femurkomponente nach den Ansprüchen (1) und (2) so konstruiert daß die Femurstielachse mit der Femurkanalachse zusammenfällt und in der Frontalebene die Collum-Centrumsachse mit der Diaphysenachse einen Winkel zwischen 125° und 145°, in aller Regel 135°, einschließt und in der axialen Aufsicht einen Winkel zwischen Diaphyse und der Schenkelhalsachse, einen sogenannten Antetorsionswinkel zwischen 5° und 15°, in aller Regel 7°, umschließt.

4. Femurkomponente nach den Ansprüchen (1) bis (3) so gestaltet, daß die Ventralfläche des proximalen metaphysären Segments (100.1) axial konvex und nach ventral in der Weise konkav gekrümmt ist, daß der Krümmungsmittelpunkt ventral liegt und der Krümmungsradius sich nach proximal kontinuierlich verkleinert.

5. Femurkomponente nach den Ansprüchen (1) bis (4) so konstruiert, daß die mediale Mantelfläche des proximalen metaphysaren Segments (100.1) axial konvex und entlang der medialen Kontur konkav gekrümmt ist und zwar in der Weise, daß der Mantelkrümmungsmittelpunkt medial liegt und sein Radius sich nach proximal kontinuierlich verkleinert.

6. Femurkomponente nach den Ansprüchen (1) bis (5) so konstruiert, dass die dorsale Mantelfläche des Stieles um die Achse herum proximal von lateral nach medial konvex-konkav-konvex (104) gekrümmt ist in Form eines Wellenschlages oder einer gerundeten "3" mit asymmetrischen Schenkeln und gerundetem Übergang.

7. Femurkomponente nach den Ansprüchen (1) bis (6) so beschaffen, dass die laterale Mantelfläche des Stieles weitgehend geradlinig, zylinderförmig konisch, nach proximal ausladend oder walzenförmig nach lateral ausladend konstruiert ist.

8. Femurkomponente nach den Ansprüchen (1) bis (7) so beschaffen, dass die Ventralfläche und/oder Medialfläche und/oder Dorsalfläche und/oder Lateralfläche des Stieles durch coaxial ausgerichtete Längswülste strukturiert ist (sind).

9. Femurkomponente nach den Ansprüchen (1) bis (8) so konstruiert, dass der Stiel über eine Schulterkomponente (200) in den Konus (300) übergeht, der für die Aufnahme einer Zuggurtung eine zentrale Bohrung aufweist.

10. Femurkomponente nach den Ansprüchen (1) bis (9) so beschaffen, daß der Konus (300) axial zu seiner Konstruktionsachse oder schräg dazu gebohrt ist für die Aufnahme einer Zuggurtung oder Zugschraube, oder dass die Schulten (200) weitere Bohrungen für die Aufnahme von weiteren Zuggurtungen (60) mit Drähten oder Seilen (71) und/oder Schub-/zugstangen und/oder Zugschrauben aufweist.

11. Femurkomponente nach den Ansprüchen (1) bis (10) so beschaffen, dass der Konus zwischen 2° und 9°, in aller Regel um 5°, in der Weise aufgerichtet ist, dass sich der CCD-Winkel nicht verändert und auch das Offset (101) unverändert bleibt, die Achse des Konus sich jedoch in die laterodorsale Zirkumferenz des kompakten Femurs projiziert 2 cm - 4 cm unterhalb des Tuberculum innominatum.

12. Femurkomponente nach den Ansprüchen (1) bis (11) so beschaffen, daß in der Schulter (200) Bohrungen für die Aufnahme von Zuggurtungen zur dorsalen, lateralen und ventralen Femurwand ausgebildet sind.

13. Femurkomponente nach Anspruch (12) so beschaffen, daß durch die Schulter in mehreren Etagenabschnitten Bohrungen für die Aufnahme von Zuggurtungen zur dorsalen, lateralen und ventralen Femurwand ausgebildet sind.

14. Femurkomponente nach den Ansprüchen (1) bis (13) so beschaffen, dass das Implantat aus Titan, Tantal, CoCrMo oder einer Legierung aus Titan oder Tantal oder aus rostfreiem Stahl hergestellt ist.

15. Femurkomponente nach den Ansprüchen (1) bis (14) so beschaffen, dass die Oberfläche der proximalen Hälfte eine Rauhigkeit von 50-250 µm, vorzugsweise 80-150 µm aufweist.

## Claims

1. A femur component of an artificial hip joint, for cement-free or cemented revision operations or primary operations, comprising a
stem which is designed of one or more parts, wherein
the stem has a dorso-lateral, cylindrical design element which is aligned axially to the marrow channel axis (130), may be solid or drilled through, and is connected to a proximal, anatomical metaphysary segment (100.1) in a fixed or assemblable manner, said segment being constructed in a conically medially eccentric manner, wherein
the proximal metaphysary segment (100.1), via a shoulder (200), carries the cone (300) for the centric or eccentric accommodation of the head, **characterised in that** the shoulder (200) comprises one or more bores (71) for receiving a tensioning strap (70).

2. A femur component according to claim 1, designed such that the cylindrical design element (103) as a continuous design shank, dorsally and laterally receives the metaphysary segment which is kidney-shaped in cross section, via a bore which is complete or not closed, and is designed as one part or several parts.

3. A femur component according to the claims 1 and 2, designed such that the femur stem axis coincides with the femur channel axis, and in the frontal plane, the Collum centre axis with the diaphysis axis encloses an angle between 125° and 145°, as a rule 135°, and in the axial plan view encloses an angle between the diaphsysis and femur neck axis, a so-called antetorsion angle between 5° and 15°, as a rule 7°.

4. A femur compoent according to one of the claims 1 to 3, designed such that the ventral surface of the proximal metaphysary segment (100.1) is curved in an axially convex manner and ventrally in a concave manner, such that the centre of curvature lies ventrally, and the radius of curvature continually reduces in the proximal direction.

5. A femur component according to the claims 1 to 4, designed such that the medial peripheral surface of the proximal metaphysary segment (100.1) is curved in a concave manner along the medial contour and specifically in a manner such that the peripheral centre of curvature lies medially and its radius continually reduces proximally.

6. A femur component according to one of the claims 1 to 5, designed such that the dorsal peripheral surface of the stem around the axis is convexly-concavely-convexly (104) curved proximally from the lateral to the medial, in the shape of a breaking wave or a rounded "3" with asymmetrical limbs and a rounded transition.

7. A femur component according to the claims 1 to 6, designed such that the lateral peripheral surface of the stem is largely designed in a straight line, cylindrically or conically cantilevered proximally or cantilevered laterally in a roller-like manner.

8. A femur component according to the claims 1 to 7, designed such that the ventral surface and/or the medial surface and/or dorsal surface and/or lateral surface of the stem is/are structured by coaxially aligned, longitudinal beads.

9. A femur component according to the claims 1 to 8, designed such that the stem via a shoulder component (200) merges into the cone (300) which comprises a central bore for receiving the tensioning strap.

10. A femur component according to the claims 1 to 9, designed such that the cone (300) is drilled axially to its design axis or obliquely thereto, for receiving a tensioning strap or tension screw, or that the shoulder (103/200) comprises further bores for receiving further tensioning straps (60) with wires or cables (71), and/or push/pull rods and/or tension screws.

11. A femur component according to one of the claims 1 to 10, designed such that the cone is directed between 2° and 9°, as a rule by 5°, in a manner such that the CCD-angle does not change or also the offset (101) remains unchanged, but that the axis of the cone projects into the lateral-dorsal circumference of the compact femur 2 cm - 4 cm below the Tuberculum innominatum.

12. A femur component according to the claims 1 to 11, designed such that bores for receiving tensioning straps to the dorsal, lateral and ventral femur wall are formed in the shoulder (200).

13. A femur component according to claim 12, designed such that bores for receiving tensioning straps to the dorsal, lateral and ventral femur wall, are formed through the shoulder in several stage sections.

14. A femur compoent according to the claims 1 to 13, designed such that the implant is manufactured of titanium, tantalum, CoCrMo or an alloy of titanium or tantalum or from stainless steel.

15. A femur according to claims 1 to 14, designed such that the surface of the proximal half has a roughness of 50-250 µm, preferably 80-150 µm.

## Revendications

1. Composant fémoral d'une articulation de la hanche artificielle pour l'opération de révision cimentée ou non ou l'opération primaire comprenant
une tige qui est configurée en une partie ou en plusieurs parties,
la tige ayant un élément de construction cylindrique dorsolatéral, lequel est aligné axialement par rapport à l'axe de canal médullaire (130), pouvant être massive ou perforée et étant reliée fixement ou de façon amovible à un segment métaphysaire anatomique proximal (100.1) qui est monté coniquement de façon médiane et excentrique,
le segment métaphysaire proximal (100.1) supportant au-dessus d'un épaulement (200) le cône (300) pour le logement de tête centrique ou excentrique, **caractérisé en ce que** l'épaulement (200) présente un ou plusieurs alésages (71) pour le logement d'une membrure de tension (70).

2. Composant fémoral selon la revendication (1) conçu de sorte que l'élément de construction cylindrique (103) en tant qu'axe de construction continu reçoit dorsalement et latéralement le segment métaphysaire réniforme en coupe transversale via un alésage complet ou non fermé, et celui-ci est conçu en une partie ou en plusieurs parties.

3. Composant fémoral selon les revendications (1) et (2) construit de sorte que l'axe de tige fémorale coïncide avec l'axe de canal médullaire, et dans le plan frontal l'axe CCD présente un angle compris entre 125° et 145°, généralement 135°, et inclut dans la vue axiale un angle cervico-diaphysaire, ce que l'on appelle un angle antétorsion, compris entre 5° et 15°, généralement 7°.

4. Composant fémoral selon les revendications (1) à (3) conçu de telle sorte que la surface ventrale du segment métaphysaire proximal (100.1) est incurvée de façon axialement convexe et de façon concave vers la partie ventrale, de sorte que le centre de courbure est ventral et le rayon de courbure se rétrécit continûment vers la partie proximale.

5. Composant fémoral selon les revendications (1) à (4) construit de telle sorte que la surface d'enveloppe médiane du segment métaphysaire proximal (100.1) est incurvée de façon axialement convexe et de façon concave le long du contour médian, et en effet de sorte que le centre de courbure de l'enveloppe est médian et son rayon se rétrécit continûment vers la partie proximale.

6. Composant fémoral selon les revendications (1) à (5) construit de sorte que la surface d'enveloppe dorsale de la tige (104) est incurvée proximalement autour de l'axe, de la partie latérale vers la partie médiane, de façon convexe-concave-convexe, sous la forme d'une vague ou d'un « 3 » arrondi avec des flancs asymétriques et une transition arrondie.

7. Composant fémoral selon les revendications (1) à (6) conçu de sorte que la surface d'enveloppe latérale de la tige est construite largement droite, cylindrique ou conique, en saillie vers la partie proximale ou en saillie cylindriquement vers la partie latérale.

8. Composant fémoral selon les revendications (1) à (7) conçu de sorte que la(les) surface(s) ventrale(s) et/ou la(les) surface(s) médiane(s) et/ou la(les) surface(s) dorsale(s) et/ou la(les) surface(s) latérale(s) de la tige est(sont) structurée(s) par le bourrelet longitudinal aligné coaxialement.

9. Composant fémoral selon les revendications (1) à (8) construit de sorte que la tige dépasse au-dessus d'un composant d'épaulement (100) dans le cône (300) qui comprend pour le logement d'une membrure de tension un alésage central.

10. Composant fémoral selon les revendications (1) à (9) conçu de sorte que le cône (300) est alésé axialement par rapport à son axe de construction ou en biais par rapport à celui-ci pour le logement d'une membrure de tension ou vis de tension, ou de sorte que l'épaulement (200) comprend d'autres alésages pour le logement d'autres membrures de tension (60) avec des fils ou des câbles (71) et/ou des tiges de poussée/traction et/ou des vis de tension.

11. Composant fémoral selon les revendications (1) à (10) conçu de sorte que le cône est élevé entre 2° et 9°, généralement 5°, de sorte que l'angle CCD demeure inchangé de même que le décalage (101), l'axe du cône se projette toutefois dans la circonférence latérodorsale du fémur compact de 2 cm à 4 cm en dessous du tubercule (tuberculus innominatus).

12. Composant fémoral selon les revendications (1) à (11) conçu de sorte que dans l'épaulement (200) sont conçus des alésages pour le logement des membrures de tension vers la paroi fémorale dorsale, latérale et ventrale.

13. Composant fémoral selon la revendication 12 conçu de sorte que dans plusieurs segments étagés sont conçus des alésages pour le logement des membrures de tension vers la paroi fémorale dorsale, latérale et ventrale.

14. Composant fémoral selon les revendications (1) à (13) conçu de sorte que l'implant est en titane, tantale, CoCrMo ou en alliage de titane et de tantale ou en acier inoxydable.

15. Composant fémoral selon les revendications (1) à (14) conçu de sorte que la surface de la moitié proximale présente une rugosité de 50 à 250 µm, de préférence de 80 à 150 µm.
